(19) Europäisches Patentamt
European Patent Office
Office européen des brevets

(11) EP 4 190 766 A1

(12) EUROPEAN PATENT APPLICATION

(43) Date of publication:
**07.06.2023 Bulletin 2023/23**

(21) Application number: **21212331.9**

(22) Date of filing: **03.12.2021**

(51) International Patent Classification (IPC):
***C05C 9/00*** $^{(2006.01)}$ ***C05C 1/02*** $^{(2006.01)}$
***C05C 3/00*** $^{(2006.01)}$ ***C05C 1/00*** $^{(2006.01)}$
***C05G 3/90*** $^{(2020.01)}$ ***C05G 5/30*** $^{(2020.01)}$

(52) Cooperative Patent Classification (CPC):
(C-Sets available)
**C05C 9/005; C05C 1/00; C05C 1/02; C05C 3/005; C05G 3/90; C05G 5/30** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SABIC Global Technologies, B.V.
4612 PX Bergen op Zoom (NL)**

(72) Inventors:
- **HEGDE, Ravi
Bangalore (IN)**
- **TANWAR, Akhilesh
Bangalore (IN)**
- **MARKANDAY, Meghna
Bangalore (IN)**
- **BELLA, Ridha
Riyadh (SA)**

(74) Representative: **Dehns
St. Bride's House
10 Salisbury Square
London EC4Y 8JD (GB)**

(54) **BENZOTHIAZOLE DERIVATIVE AS A UREASE INHIBITOR FOR FERTILIZER APPLICATION










**

(57) Disclosed herein are fertilizer compositions comprising: a nitrogen-based fertilizer; and a benzothiazole derivative represented by the general Formula (I). The nitrogen-based fertilizer and benzothiazole derivative are comprised of a melt admixture or the nitrogen-based fertilizer comprises granules and the benzoquinone derivative is coated on the granules. A soil treated with the fertilizer composition exhibits a nitrogen loss of less than 20% over a 21-day period when measured via ammonia volatilization of the treated soil.

EP 4 190 766 A1

(52) Cooperative Patent Classification (CPC): (Cont.)

C-Sets
**C05C 1/02, C05C 9/005;**
**C05C 3/005, C05C 9/005**

## Description

### TECHNICAL FIELD

The present disclosure relates to fertilizer compositions, more specifically to fertilizer compositions including a benzothiazole derivative.

### BACKGROUND

Fertilizers have long been used to provide nitrogen to soil to enhance plant growth. The most widely used and agriculturally important nitrogen fertilizer is urea, $CO(NH_2)_2$. Most urea currently produced is used as a fertilizer in its granular (or prilled) form. After application of urea to soil, it is readily hydrolyzed to yield ammonia and carbon dioxide. This process is catalyzed by the enzyme urease, which is produced by some bacteria and fungi that may be present in soil. The gaseous products formed by the hydrolysis reaction (namely, ammonia and carbon dioxide) volatilize to the atmosphere. Thus, the soil may lose a substantial amount of nitrogen via these processes. Attempts to reduce losses of applied nitrogen have conventionally included the application of urease inhibitors and/or nitrification inhibitors as additives to fertilizer. Examples of urease inhibitors include thiophosphoric triamide compounds disclosed in U.S. Patent No. 4,530,714 to Kolc et al., which is incorporated herein by reference. Thiophosphoric triamide compounds include N-(n-butyl) thiophosphoric triamide (NBTPT), the most developed representative of this class of compounds. However, NBTPT effectiveness may be limited in acidic conditions and is generally sensitive to temperature both in fertilizer and in soil. See, for example, Soil Use & Management, 2008, 24, 246; Soil Sci. Soc. Am. J., 2015; Soil Res., 2011, 49, 315. Therefore, there remains a need to identify a novel urease inhibitor that can be effective at wider pH and temperature ranges.

These and other shortcomings are addressed by aspects of the present disclosure.

### BRIEF DESCRIPTION OF FIGURES

The accompanying figures, which are incorporated in and constitute a part of this specification, illustrate several aspects and together with the description serve to explain the principles of the disclosure.

FIGS. 1A and 1B present the %BZT recovered at 1-, 13-, 84-, 120-, 150-, and 180-day intervals for samples stored at ambient and controlled conditions, respectively.

FIGS. 2A and 2B are molecular modeling simulations showing a *Sporosarcina Pasteurii* Urease structure and enhanced image of the optimized urease active site, respectively.

### SUMMARY

The present disclosure relates to a fertilizer composition comprising: a nitrogen-based fertilizer; and a urease inhibitor comprising a benzothiazole derivative represented by general Formula (I).

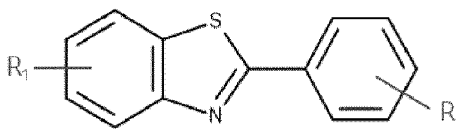

Formula I

wherein R and $R_1$ are independently selected from the group consisting of H, OH, OMe, OEt, $NO_2$, $NH_2$, NHMe, $NH(Me)_2$, SH, F, Cl, Br, I, and CN, wherein the nitrogen-based fertilizer and benzothiazole derivative are comprised of a melt admixture or wherein the nitrogen-based fertilizer comprises granules and the benzothiazole derivative is coated on the granules. A soil treated with the fertilizer composition exhibits a nitrogen loss of less than 20% over a 21-day period when measured via ammonia volatilization assays of the treated soil.

Further aspects of the present disclosure relate to methods of forming the disclosed fertilizer composition.

### DETAILED DESCRIPTION

It is well known in the art to use urea and urea compositions in fertilizers, for application to the soil. The effective life of such fertilizers, however, is of short duration wherever microbiological activity exists in the soil to which the fertilizer is applied. Upon soil application of urea, it hydrolyzes to ammonia and carbamic acid by the action of urease enzyme.

Typically, the ammonia escapes to the atmosphere unless it reacts with water to form a plant absorbable form, ammonium ($NH^{4+}$). The urease catalyzed hydrolysis is a rapid process providing nitrogen in the form of ammonia, which may be harmful to the environment. Attack by the urease enzyme causes urea to degrade to carbon dioxide and ammonia. While the conversion of urea to ammonia and oxidation of ammonia to nitrates within the soil is beneficial to plants, conversions occurring on top of the soil, where fertilizers are applied, also results in a loss of nitrogen. Christianson, C.B., et al. (Soil Biology and Biochemistry, 1993, 1107) reported complete hydrolysis of urea within two days of application in highly alkaline soil (namely, a pH of about 8.2 or higher). In acidic soils (about pH 5.2), the urea took twice as long to hydrolyze. Maintaining a sufficient level of nitrogen concentration in the soil may also be difficult over time because nitrogen and nitrogen containing compounds (such as urea) are water soluble. When rain or water run-off contacts the soil, the nitrogen or nitrogen containing compounds may be carried with the water to surrounding waterways - ultimately, a water and atmospheric pollutant.

Conventional solutions to reduce nitrogen loss include urease inhibitors and/or nitrification inhibitors as additives to fertilizer. Urease inhibitors are generally compounds capable of inhibiting the catalytic activity of the urease enzyme on urea in the soil. Nitrification inhibitors refer to compounds capable of inhibiting the bacterial oxidation of ammonium to nitrate in the soil. Urease inhibitors and nitrification inhibitors may be combined with fertilizers in various ways. They may be coated onto fertilizer granules or mixed into fertilizer matrices. A common urease inhibitor is N-(n-butyl) thiophosphoric triamide (NBTPT or NBTPT), which may be incorporated into a urea-containing fertilizer. NBTPT may reduce the rate at which urea is hydrolyzed in the soil to ammonia. NBTPT is believed however to degrade at elevated temperatures into compounds that may not provide the desired inhibitory effects on the urease enzyme. Accordingly, its combination with other solid materials to provide a material capable of inhibiting urease, particularly via granulation with urea (which generally employs heat) may be challenging. As such, there is a need for urease inhibitor-containing compositions that can be combined with urea, desirably using current urea manufacturing practices, so as to produce fertilizer compositions that provide effective urease inhibition.

Fertilizer compositions of the present disclosure may slow nitrogen loss in treated soil by inhibiting urease activity at levels comparable to that of NBTPT. The disclosed compositions may comprise a nitrogen-based fertilizer, a benzothiazole derivative represented by general Formula I:

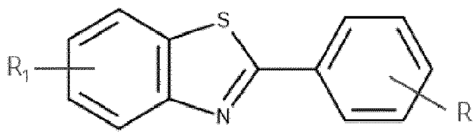

Formula I

Urease Inhibition

Urea hydrolyses rapidly when placed under or on the surface of soil containing urease, a crystallizable enzyme present in numerous bacteria and fungi. Urease catalyzes the conversion of urea into ammonia and carbon dioxide. The relevant reactions may proceed as shown below:

$$CO(NH_2)_2 + 2H_2O \xrightarrow{\text{urease}} 2NH_3 + H_2CO_3$$

$$NH_3 + H_2O \rightarrow NH_4^+ + OH^-$$

The ammonia formed may be held in the soil and is available to the plant as a nutrient. However, as described herein, a large amount of the ammonia may be lost to the air, and accumulated ammonium species in the soil may damage germinating seedlings and/or young plants. Urease inhibition may thus refer to a reduction, or otherwise slowing down, of the conversion of urea to ammonium ($NH_4^+$) in soil by impeding urease. As noted herein, examples of urease inhibitors configured to reduce the rate of urea hydrolysis include various phosphoro-compounds such as N-(n-butyl) thiophosphoric triamide (NBTPT). However, NBTPT effectiveness is limited at acidic condition and sensitive to temperature.

Fertilizer compositions of the present disclosure provide successful urease inhibition comparable to that of NBTPT, however under acidic conditions and at both ambient and controlled temperature conditions, as well as varying moisture levels or humidity.

Benzothiazole Derivative and Urea Fertilizer Compositions

In various aspects, the fertilizer compositions of the present disclosure combine a nitrogen-based fertilizer, such as urea, with a urease inhibitor comprising a benzothiazole derivative as presented in Formula I.

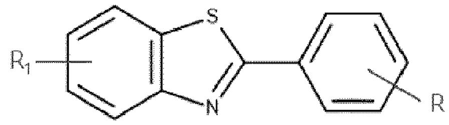

Formula I

R and R1 may be independently selected from the group consisting of H, OH, OMe, OEt, $NO_2$, $NH_2$, NHMe, $NH(Me)_2$, SH, F, Cl, Br, I, and CN. In certain aspects, R is a hydroxy group.

As a specific example, the composition may comprise a urea, and a urease inhibitor comprising a benzothiazole derivative represented by Formula II, namely 2-(3-hydroxyphenyl)benzothiazole.

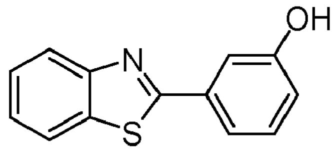

Formula II

Nitrogen-based fertilizer as used herein is described broadly and may refer to a fertilizer comprising nitrogen. More specifically, and as described further herein, the nitrogen-based fertilizer may comprise a urea. Accordingly, urea may include urea-based fertilizers such as, but not limited to, urea ammonium sulfate, urea ammonium nitrate, or a combination thereof.

The nitrogen-based fertilizer and benzothiazole derivative may comprise a melt admixture as the fertilizer composition. A melt admixture may be formed via a process of combining the nitrogen-based fertilizer, such as urea, and a benzothiazole derivative, such as Formula I, at a temperature of at least the melting temperature of the nitrogen-based fertilizer. The benzothiazole derivative in the melt admixture may have a concentration of from about 300 ppm to about 3000 ppm. As a specific example, the benzothiazole derivative in the melt admixture may have a concentration of about 1000 ppm.

In yet further aspects, the disclosed fertilizer composition is comprised of a granulated nitrogen-based fertilizer having the benzoquinone derivative coated thereupon. That is the composition may comprise the nitrogen-based fertilizer in a granule form having the benzothiazole derivative coated on the granules.

Further provided herein are adducts of the fertilizer composition comprising the nitrogen-based fertilizer and benzothiazole derivative represented by Formula I. The adducts provided as-formed, may be provided in combination with one or more other components, such as additional urease inhibitor or a fertilizer composition, for example, in the form of a nitrogen source including, but not limited to, a urea source. That is, the fertilizer composition may include one or more additives. These additives may include, but are not limited to, an additional fertilizer, a micronutrient, a secondary nutrient, or an organic additive. In some instances, the fertilizer composition can include one or more of iron, manganese, zinc, and copper. In some instances, the fertilizer composition includes MgO.

The compositions disclosed herein can, in some aspects, exhibit novel, slow-release of the one or more urease inhibitors. Thus, the fertilizer composition may be in the form of an adduct wherein the adduct comprises the fertilizer composition and one or more known urease inhibitors. For example, an adduct may comprise the fertilizer composition and one or more of phenyl phosphorodiamidate (PPDA), N-(n-butyl) thiophosphoric triamide (NBTPT), and thiourea. In yet further aspects, the fertilizer composition may be an adduct further comprising one or more of a urease inhibitor and nitrification inhibitors. Suitable nitrification inhibitors may comprise dicyandiamide (DCD, $C_2H_4N_4$), 3,4-dimethylpyrazole phosphate (DMPP), 2-chloro-6-(trichloromethyl)-pyridine (nitrapyrin), 5-ethoxy-3-trichloromethyl -1, 2, 4-thiadiazol (terrazole), 2-Amino-4-chloro-6-methylpyrimidine (AM), 2-mercapto-benzothiazole (MBT), or 2-sulfanimalamidothiazole (ST), or any combination thereof.

**Applications and Benefits**

In various aspects, the present disclosure provides a method of reducing hydrolysis of urea to ammonia in soil. The method may comprise applying to a soil a fertilizer composition comprising a nitrogen-based fertilizer and a benzothiazole derivative represented by general Formula I:

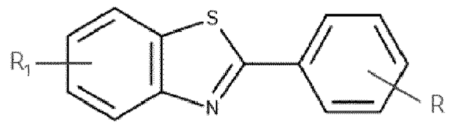

Formula I

R and R1 may be independently selected from the group consisting of H, OH, OMe, OEt, $NO_2$, $NH_2$, NHMe, $NH(Me)_2$, SH, F, Cl, Br, I, and CN. In certain aspects, R is a hydroxy group

A soil treated with the fertilizer composition may exhibit a nitrogen loss of less than 25%, less than 20%, or less than 15% over at least a 21-day period when measured via ammonia volatilization of the treated soil. Accordingly, the application of the disclosed fertilizer composition to a given crop may encourage the following:

- delayed urea hydrolysis resulting in nutrient nitrogen is available to the plant over a longer period of time;
- reduced build-up of ammonia in the soil following the application of the fertilizer composition;
- reduced potential for nitrogen loss through ammonia volatilization;
- reduced potential for damage to seedlings and young plants from exposure to excessive ammonia
- plant uptake of nitrogen is increased; and, ultimately
- increased crop yield.

The disclosed fertilizer composition may be suitable for a variety of crops/plants, including but not limited to vegetables, corn, potatoes, onions, grass (lawns), gardens, flowers, shrubs and hedges.

**Methods for forming the fertilizer compositions**

Aspects of the disclosure further relate to methods for making BZT-coated urea fertilizer or a urea-BZT melt-admixture. For BZT-coated urea, urea granules or particles may be coated with a concentrated amount of BZT.

The BZT-coated urea fertilizer may be prepared by any suitable manner known in the art. As an example, BZT may be ground to a desirable particle size to promote coating adhesion under ambient conditions. For example, BZT may be ground to particles having an average particle size less than 350 microns, less than 300 microns, less than 250 microns, or less than 225 microns. The ground BZT may be combined with granulated urea and continuously mixed. A coating solution may be introduced to promote adhesion of the BZT to the urea granules. As a further example, the BZT may be combined with the urea granules as a coating composition. The coating may be applied via any suitable manner known in the art, such as for example, via deposition in a fluidized bed.

The urea-BZT melt admixture formulation may be prepared according to any suitable manner known in the art. In a specific example, the admixture fertilizer composition may be formed according to a cost effective and simple process. In a specific aspect, a method of forming an admixture fertilizer composition comprises heating urea to form a urea melt. Formation of the urea melt may comprise heating the urea to a temperature of at least 130 °C.

The urea melt may be combined with a benzothiazole compound of Formula I (or Formula II as described herein) to provide a melt mixture. Combining may proceed at a temperature about or greater than the temperature required to form the urea melt. For example, the combining may proceed at a temperature of from about 130 °C to about 135 °C. An additive, such as an additional urease inhibitor, nitrification inhibitor, or further additive, may be added to the melt mixture prior to the granulating step. In some examples, the melt mixture comprising the urea melt and benzothiazole compound may be combined with a solvent. Suitable solvents may include, but are not limited to, propylene glycol, N-methyl-2-pyrrolidone, dimethyl sulfoxide, dimethylformamide, dimethylacetamide, xylene.

The obtained melt mixture may be granulated to provide the disclosed fertilizer composition. A number of granulation methods are known, including falling curtain, spheroidization-agglomeration drum granulation, prilling and fluid bed granulation technologies. Any of the foregoing granulation methods may be suitable.

ASPECTS

In various aspects, the present disclosure pertains to and includes at least the following aspects.

Aspect 1. A fertilizer composition comprising: a nitrogen-based fertilizer; and urease inhibitor comprising a benzothiazole derivative represented by the general Formula (I);

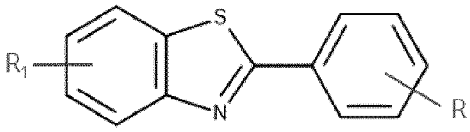

Formula I,

wherein R and $R_1$ are independently selected from the group consisting of H, OH, OMe, OEt, $NO_2$, $NH_2$, NHMe, $NH(Me)_2$, SH, F, Cl, Br, I, and CN, and wherein the nitrogen-based fertilizer and benzothiazole derivative are comprised of a melt admixture or wherein the nitrogen-based fertilizer comprises granules and the benzothiazole derivative is coated on the granules.

Aspect 2. The fertilizer composition of aspect 1, wherein a soil treated with the fertilizer composition exhibits a nitrogen loss of less than 20% over a 21-day period when measured via ammonia volatilization of the treated soil.

Aspect 3. The fertilizer composition of any one of aspects 1-2, wherein R is a hydroxy group.

Aspect 4. The fertilizer composition of any one of aspects 1-2 wherein $R_1$ is hydrogen.

Aspect 5. The fertilizer composition of any one of aspects 1-2, wherein R is a hydroxy group and $R_1$ is hydrogen.

Aspect 6. The fertilizer composition of any one of aspects 1-5, wherein the nitrogen-based fertilizer is granulated and comprises a coating, wherein the coating comprises the benzothiazole derivative of Formula I.

Aspect 7. The fertilizer composition of any one of aspects 1-5, wherein the fertilizer composition is a melt admixture comprising the nitrogen-based fertilizer and the benzothiazole derivative represented by Formula (I).

Aspect 8. The fertilizer composition of aspect 7, wherein the melt admixture is formed via a process of combining the nitrogen-based fertilizer and the benzothiazole derivative of Formula I at a temperature of at least a melting temperature of the nitrogen-based fertilizer.

Aspect 9. The fertilizer composition of any one of aspects 7-8, wherein the benzothiazole derivative in the melt admixture has a concentration of from about 100 ppm to about 3000 ppm.

Aspect 10. The fertilizer composition of any one of aspects 7-8, wherein the benzothiazole derivative in the melt admixture has a concentration of about 1000 ppm.

Aspect 11. The fertilizer composition of any one of aspects 1-10, wherein the nitrogen-based fertilizer comprises urea, urea ammonium sulfate, urea ammonium nitrate, or a combination thereof.

Aspect 12. The fertilizer composition of any one of aspects 1-11, wherein the fertilizer composition further comprises a urease inhibitor.

Aspect 13. The fertilizer composition of aspect 12, wherein the urease inhibitor comprises phenyl phosphorodiamidate (PPDA), N-(n-butyl) thiophosphoric triamide (NBTPT), thiourea, or a combination thereof.

Aspect 14. The fertilizer composition of any one of aspects 1-13, wherein the fertilizer composition further comprises a nitrification inhibitor.

Aspect 15. The fertilizer composition of aspect 14, wherein the nitrification inhibitor comprises: dicyandiamide (DCD, $C_2H_4N_4$); 3,4-dimethylpyrazole phosphate (DMPP); 2-chloro-6-(trichloromethyl)-pyridine (nitrapyrin); 5-ethoxy-3-trichloromethyl -1, 2, 4-thiadiazol (terrazole); 2-Amino-4-chloro-6-methylpyrimidine (AM); 2-mercapto-benzothiazole

(MBT); 2-sulfanimalamidothiazole (ST); or a combination thereof.

Aspect 16. A fertilizer composition comprising: a melt admixture comprising: a urea; and a benzothiazole derivative represented by Formula II

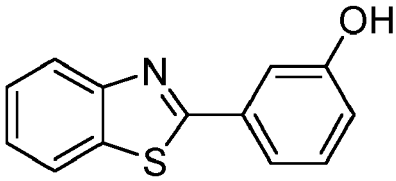

Formula II,

wherein a soil treated with the fertilizer composition exhibits a nitrogen loss of less than 20% over a 21-day period when measured via ammonia volatilization of the treated soil.

Aspect 17. The fertilizer composition of aspect 16, wherein the melt admixture is formed via a process of combining the urea and the compound of Formula II at a temperature of at least a melting temperature of the nitrogen-based fertilizer.

Aspect 18. The fertilizer composition of any one of aspects 16-17, wherein the fertilizer composition comprises at least 95 wt. % urea.

Aspect 19. The fertilizer composition of any one of aspects 16-17, wherein the fertilizer composition comprises at least 98 wt. % urea.

Aspect 20. The fertilizer composition of any one of aspects 16-19, wherein the composition comprises from about 100 ppm to about 3000 ppm of the benzothiazole derivative.

Aspect 21. A method of forming a fertilizer composition, the method comprising: heating urea to form a urea melt; combining the urea melt with a benzothiazole compound of Formula I to provide a melt mixture;

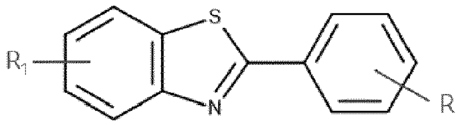

Formula I,

wherein R and $R_1$ are independently selected from the group consisting of H, OH, OMe, OEt, $NO_2$, $NH_2$, NHMe, $NH(Me)_2$, SH, F, Cl, Br, I, and CN, and granulating the melt mixture to provide the fertilizer composition.

Aspect 22. The method of aspect 21, further comprising adding an additive to the melt mixture prior to granulating.

Aspect 23. The method of any one of aspects 21-22, wherein step (b) of combining the urea melt with a compound of Formula I further includes combining the melt mixture with a solvent.

Aspect 24. The method of any one of aspects 21-23, wherein step (b) is performed at a temperature of 130 °C to 135 °C.

Aspect 25. The method of any one of aspects 21-24, wherein the additive comprises a urease inhibitor, a nitrification inhibitor, or a combination thereof.

Aspect 26. The method of any one of aspects 21-25, wherein step (a) of heating the urea to form a urea melt comprises heating the urea to a temperature of at least 130 °C.

Aspect 27. A fertilizer composition comprising: a nitrogen-based fertilizer; and a urease inhibitor comprise of benzothiazole derivative represented by the general Formula (I);

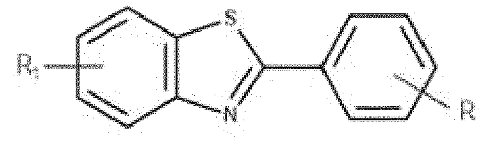

Formula I

wherein R and $R_1$ are independently selected from the group consisting of H, OH, OMe, OEt, $NO_2$, $NH_2$, NHMe, $NH(Me)_2$, SH, F, Cl, Br, I, and CN, and wherein the nitrogen-based fertilizer and benzothiazole derivative are comprised of a melt admixture or wherein the nitrogen-based fertilizer comprises granules and the benzothiazole derivative is coated on the granules.

Aspect 28. The fertilizer composition of aspect 27, wherein a soil treated with the fertilizer composition exhibits a nitrogen loss of less than 20% over a 21-day period when measured via ammonia volatilization of the treated soil.

Aspect 29. A fertilizer composition comprising: a nitrogen-based fertilizer; and a benzothiazole derivative represented by the general Formula (I);

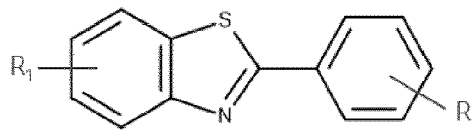

Formula I,

wherein R and $R_1$ are independently selected from the group consisting of H, OH, OMe, OEt, $NO_2$, $NH_2$, NHMe, $NH(Me)_2$, SH, F, Cl, Br, I, and CN, and wherein the nitrogen-based fertilizer and benzothiazole derivative are comprised of a melt admixture or wherein the nitrogen-based fertilizer comprises granules and the benzothiazole derivative is coated on the granules, wherein a soil treated with the fertilizer composition exhibits a nitrogen loss of less than 20% over a 21 - day period when measured via ammonia volatilization of the treated soil.

## EXAMPLES

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature), but some errors and deviations should be accounted for. Unless indicated otherwise, parts are parts by weight, temperature is in °C or is at ambient temperature, and pressure is at or near atmospheric. Unless indicated otherwise, percentages referring to composition are in terms of wt.%.

There are numerous variations and combinations of mixing conditions, for example, component concentrations, extruder design, feed rates, screw speeds, temperatures, pressures and other mixing ranges and conditions that can be used to optimize the product purity and yield obtained from the described process. Only reasonable and routine experimentation will be required to optimize such process conditions.

*Synthesis of 2-(3-Hydroxyphenyl)benzothiazole (BZT).* An amount of sodium bisulfite (1.67 g, 16 mmol) was added to dimethylacetamide (5 mL) and stirred for 10 minutes. An amount of 2-aminothiophenol (1 g, 8 mmol) and 3-hydroxy benzaldehyde (1.07 g, 8.8 mmol) were added into the reaction mixture. The reaction mixture was heated for 6 hours at 120 °C and the completion of the reaction was confirmed via thin layer chromatography TLC (hexane: ethylacetate, 8:2). The reaction mass was cooled to room temperature and then poured into cold water under stirring. The solid was filtered and the product was recrystallized in dichloromethane to yield a pale-yellow product in 61% yield. The product was confirmed via $^{1}H$- & $^{13}C$- NMR and liquid chromatography - mass spectrometry LC-MS analysis and data is in accordance with the literature (RSC. Adv., 2015, 5, 28814).

*Formation of BZT coated urea (BZT-CS).* BZT was ground, sieved and selected particles smaller than 212 microns for better adhesion on urea granules. An amount of 200 mg of inhibitor powder (BZT) was added slowly on 200 grams of urea granules in a glass bottle while it is continuously rotating manually. Mixing continued for additional 5 minutes. A 200 microliter coating solution (propylene glycol: dimethylsulfoxide DMSO (2:1), for better adhesion of inhibitor) was added dropwise and mixed thoroughly before packing. As a control experiment, only the coating solution was used without inhibitor. Uniformity of coating was confirmed by a separate experiment wherein 10 mg/mL indigo carmine (IC) dye was used along with coating solution resulting in colored coated urea granules.

*BZT exposed to urea melt condition (BZT-MS).* To mimic the commercial granulation condition, powdered

inhibitor (0.1% wt/wt) was added to melted urea with 2% water and maintained for 15 minutes at about 132 °C in an oil bath. Then the molten mass containing inhibitor was poured into a glass tray, cooled and cut to get flakes, which was used for further studies.

*Long-term storage stability study.* BZT coated sample (BZT-CS) and exposed to urea melt condition sample (BZT-MS) were stored at ambient condition and under controlled condition (switching between storage at 40 °C, 75% relative humidity (RH) and 30 °C, 50% RH every 12 hours alternately) for this study. Samples were taken at different time intervals for BZT recovery analysis by HPLC method. Data recorded until 180 days and shown in FIGS. 1A and 1B.

*Storage stability.* The inhibitor BZT was found to be stable in both the samples. At least 90.7% BZT recovery was observed at any given point of time and at both the conditions tested (ambient and controlled). A comparative inhibitor NBTPT was found to be unstable under similar storage conditions. See, (detailed study published in *Soil Use and Management,* 2008, 24, 246). BZT has thus exhibited better thermal stability with urea when compared to NBTPT.

*Ammonia volatilization.* Ammonia volatilization study was conducted for two BZT coated samples and compared with a standard NBTPT coated urea sample. Tested formulation details provided in Table 1 below. BZT-1 and BZT-2 differ in the quantity of BZT coated on urea. BZT-1 has the standard quantity used for benchmark NBTPT.

**Table 1**. Formulations for ammonia volatilization.

| Sample | Estimated amount, PPM | Quantity, millimole (mmol)/ 100 g urea |
|---|---|---|
| BZT-1 | 1290 | 5.7 |
| BZT-2 | 690 | 3.0 |
| NBTPT | 960 | 5.7 |

Alkaline sandy soil (pH 7.8) was used to quantify the ammonia volatilization losses. The nitrogen (N) sources (BZT-1, BZT-2, and NBTPT urea formulations with control urea) were applied at a rate of 0.830 g N (the equivalent of 200 kg N ha-1) to 7 kg soil in volatilization pots with an inner diameter of 23 centimeters. Five replicates were used for each treatment. The N sources were uniformly applied to the soil surface. The soil moisture content throughout the volatilization period was maintained at 75% of field moisture capacity. The pots were sealed tight with air inflow from a compressed air system and outflow to acid traps. Air was passed through an acid scrubber to remove any $NH_3$ from air and humidified before entering the pots. The airflow rate was adjusted to 5 L $min^{-1}$ for each pot to allow for complete mixing and maximum removal of any ammonia formed. All ammonia (outflow from the pots) was captured in phosphoric acid traps. The traps were sampled and analyzed for ammoniacal N at regular intervals at 1, 3, 4, 7, 14 and 21 days after fertilization. The results are presented as cumulative ammonia loss in Table 2.

**Table 2.** Ammonia volatilization data over 21 days.

| Sample | Cumulative $NH_3$ loss over 21 days, % |
|---|---|
| Urea control | 32 |
| NBTPT | 6 |
| BZT-1 | 13 |
| BZT-2 | 23 |

As shown, un-treated urea lost nearly a third of its nitrogen. BZT treated samples exhibited much lower nitrogen loss, comparable to the loss shown by NBTPT treated samples (although greater).

*Molecular modeling.* Molecular modeling was used to estimate the relative binding affinity of the Benzothiazole (BZT) as compared to the NBTPT molecule in the active site of urease inhibitor. Method used here is to develop active site model of urease and then study interaction of different inhibitors. *Sporosarcina Pasteurii* Urease (SPU) enzyme structure (5OL4) was obtained from protein data bank. The active site of SPU enzyme comprising αHis222, carboxylated αLys220, αHis249, αHis275, αHis323, αHis137, αHis139, αAsp363 and αala366, along with both Nickel atoms and three oxygen atoms connected to nickel was extracted, Hydrogen atoms were added to satisfy valency of different atoms and overall charge of +1.0 was set on the system to obtain closed shell configuration. Constrained geometry optimization, at PBE0/def2-SVP level, was carried out by freezing all non-Hydrogen atoms except bridged OH group and two water molecules attached to Nickel atoms. FIGS. 2A and 2B shows the SPU structure and the optimized Active site structure.

For benchmarking active site structure, three conventional inhibitors reported earlier (J. Agric. Food Chem. 2008, 56, 3721.) were introduced at the urease active site followed by constrained geometry optimization at PBE0/def2-SVP level, by freezing all non-Hydrogen atoms except bridged OH group and two water molecules attached to Nickel

atoms and the inhibitor molecule. Binding energy (BE) of the inhibitor with active site was estimated by using equation (1).

$$BE = Energy(Enzyme\text{-}Inhibitor\ complex) - [Energy(Active\ site) + Energy\ (Inhibitor)] \quad (1)$$

During competitive inhibition, the inhibitor molecule, which has higher binding energy with the enzyme active site, would be a better inhibitor and hence would have lower $IC_{50}$ values. After establishing the validity of the active site model, BE was estimated for BZT using the same method described above. Binding energy estimations are shown in Table 3. The binding energy of BZT (-23.3 kcal/mol) was close to the BE of NBTPT, suggesting the inhibiting nature of BZT.

**Table 3**. Calculated binding energy from molecular modeling.

| Inhibitor | BE(S1) [Kcal/mol] | $IC_{50}$ [nM]* |
|---|---|---|
| 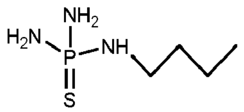 | -27.4 | 100 |
| 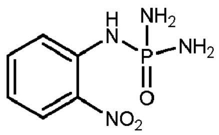 | -34.2 | 3 |
| 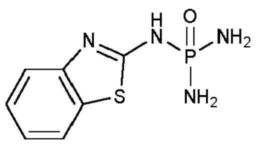 | -34.9 | 2 |
| 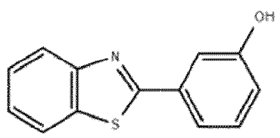 | -23.3 | |

Any publications mentioned herein are incorporated herein by reference to disclose and describe the methods and/or materials in connection with which the publications are cited.

It is also to be understood that the terminology used herein is for the purpose of describing particular aspects only and is not intended to be limiting. As used in the specification and in the claims, the term "comprising" may include the aspects or aspects "consisting of' and "consisting essentially of." Unless defined otherwise, all technical and scientific terms used herein have the same meaning as commonly understood by one of ordinary skill in the art to which this invention belongs. In this specification and in the claims which follow, reference will be made to a number of terms which shall be defined herein.

As used in the specification and the appended claims, the singular forms "a," "an" and "the" include plural referents unless the context clearly dictates otherwise. Thus, for example, reference to "a glass fiber" includes mixtures of two or more such glass fibers.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit falling within a range between two particular units are also disclosed. For example, if 10 and 15 are disclosed, then 11, 12, 13, and 14 are also disclosed.

As used herein, the terms "optional" or "optionally" mean that the subsequently described event, condition, component, or circumstance may or may not occur, and that the description includes instances where said event or circumstance occurs and instances where it does not.

As used herein, the term or phrase "effective," "effective amount," or "conditions effective to" refers to such

amount or condition that is capable of performing the function or property for which an effective amount is expressed. As will be pointed out below, the exact amount or particular condition required may vary from one aspect or aspect to another, depending on recognized variables such as the materials employed and the processing conditions observed. Thus, it is not always possible to specify an exact "effective amount" or "condition effective to" for each aspect or aspect encompassed by the present disclosure. However, it should be understood that an appropriate effective amount or condition effective to achieve a desired results will be readily determined by one of ordinary skill in the art using only routine experimentation.

Disclosed are the components to be used to prepare disclosed compositions of the invention as well as the compositions themselves to be used within methods disclosed herein. These and other materials are disclosed herein, and it is understood that when combinations, subsets, interactions, groups, etc. of these materials are disclosed that while specific reference of each various individual and collective combinations and permutation cannot be explicitly disclosed, each is specifically contemplated and described herein. This concept applies to all aspects of this application including, but not limited to, steps in methods of making and using the compositions of the invention. Thus, if there are a variety of additional steps that can be performed it is understood that each of these additional steps can be performed with any specific aspect or combination of aspects of the methods of the invention.

References in the specification and concluding claims to parts by weight, of a particular component in a composition or article, denotes the weight relationship between the element or component and any other elements or components in the composition or article for which a part by weight is expressed. Thus, in a composition containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included. For example, if a particular element or component in a composition or article is said to have 8% weight, it is understood that this percentage is relation to a total compositional percentage of 100%.

Each of the component starting materials disclosed herein are either commercially available and/or the methods for the production thereof are known to those of skill in the art.

It will be apparent to those skilled in the art that various modifications and variations can be made in the present invention without departing from the scope or spirit of the invention. Other aspects of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A fertilizer composition comprising:

   a nitrogen-based fertilizer; and
   a urease inhibitor comprising a benzothiazole derivative represented by the general Formula (I);

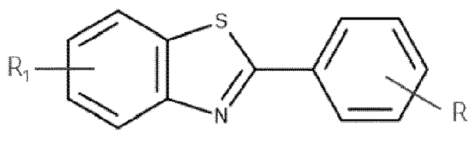

Formula I

   wherein R and $R_1$ are independently selected from the group consisting of H, OH, OMe, OEt, $NO_2$, $NH_2$, NHMe, $NH(Me)_2$, SH, F, Cl, Br, I, and CN,
   and
   wherein the nitrogen-based fertilizer and benzothiazole derivative are comprised of a melt admixture or wherein the nitrogen-based fertilizer comprises granules and the benzothiazole derivative is coated on the granules,

2. The fertilizer composition of claim 1, wherein a soil treated with the fertilizer composition exhibits a nitrogen loss of less than 20% over a 21-day period when measured via ammonia volatilization of the treated soil.

3. The fertilizer composition of any one of claims 1-2, wherein R is a hydroxy group.

4. The fertilizer composition of any one of claims 1-3, wherein the nitrogen-based fertilizer is granulated and comprises

a coating, wherein the coating comprises the benzothiazole derivative of Formula I.

**5.** The fertilizer composition of any one of claims 1-3, wherein the fertilizer composition is a melt admixture comprising the nitrogen-based fertilizer and the benzothiazole derivative represented by Formula (I).

**6.** The fertilizer composition of claim 5, wherein the melt admixture is formed via a process of combining the nitrogen-based fertilizer and the benzothiazole derivative of Formula I at a temperature of at least a melting temperature of the nitrogen-based fertilizer.

**7.** The fertilizer composition of any one of claims 5-6, wherein the benzothiazole derivative in the melt admixture has a concentration of from about 100 ppm to about 3000 ppm.

**8.** The fertilizer composition of any one of claims 5-7, wherein the benzothiazole derivative in the melt admixture has a concentration of about 1000 ppm.

**9.** The fertilizer composition of any one of claims 1-8, wherein the nitrogen-based fertilizer comprises urea, urea ammonium sulfate, urea ammonium nitrate, or a combination thereof.

**10.** The fertilizer composition of any one of claims 1-9, wherein the urease inhibitor further comprises phenyl phosphorodiamidate (PPDA), N-(n-butyl) thiophosphoric triamide (NBTPT), thiourea, or a combination thereof.

**11.** The fertilizer composition of any one of claims 1-10, wherein the fertilizer composition further comprises a nitrification inhibitor.

**12.** The fertilizer composition of claim 11, wherein the nitrification inhibitor comprises: dicyandiamide (DCD, $C_2H_4N_4$); 3,4-dimethylpyrazole phosphate (DMPP); 2-chloro-6-(trichloromethyl)-pyridine (nitrapyrin); 5-ethoxy-3-trichloromethyl -1, 2, 4-thiadiazol (terrazole); 2-Amino-4-chloro-6-methylpyrimidine (AM); 2-mercapto-benzothiazole (MBT); 2-sulfanimalamidothiazole (ST); or a combination thereof.

**13.** A fertilizer composition comprising:
a melt admixture comprising:

a urea; and
a benzothiazole derivative represented by Formula II

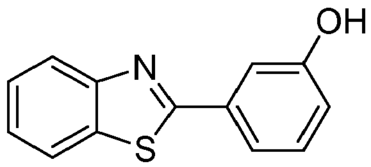

Formula II

wherein a soil treated with the fertilizer composition exhibits a nitrogen loss of less than 20% over a 21-day period when measured via ammonia volatilization of the treated soil.

**14.** A method of forming a fertilizer composition, the method comprising:

a. heating urea to form a urea melt;
b. combining the urea melt with a benzothiazole compound of Formula I to provide a melt mixture;

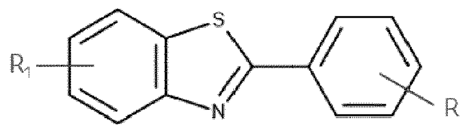

Formula I

wherein R and $R_1$ are independently selected from the group consisting of H, OH, OMe, OEt, $NO_2$, $NH_2$, NHMe, $NH(Me)_2$, SH, F, Cl, Br, I, and CN, and

c. granulating the melt mixture to provide the fertilizer composition.

**15.** The method of claim 14, further comprising adding an additive to the melt mixture prior to granulating.

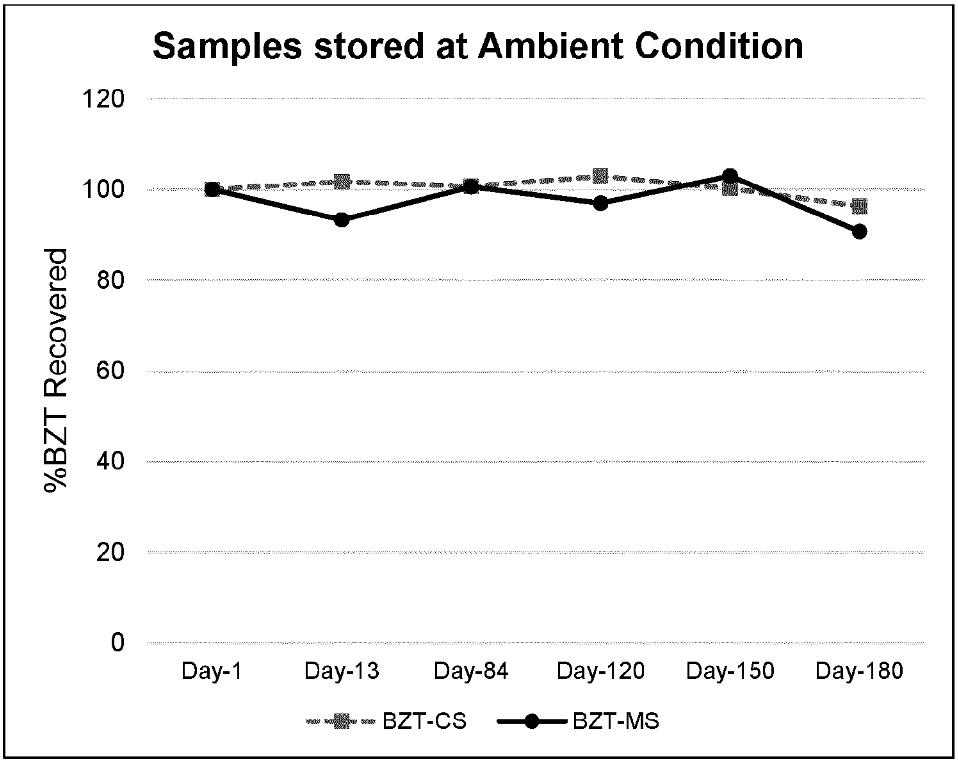
Samples stored at Ambient Condition
120
100
80
60
40
20
0
%BZT Recovered
Day-1
Day-13
Day-84
Day-120
Day-150
Day-180
BZT-CS
BZT-MS

FIG. 1A

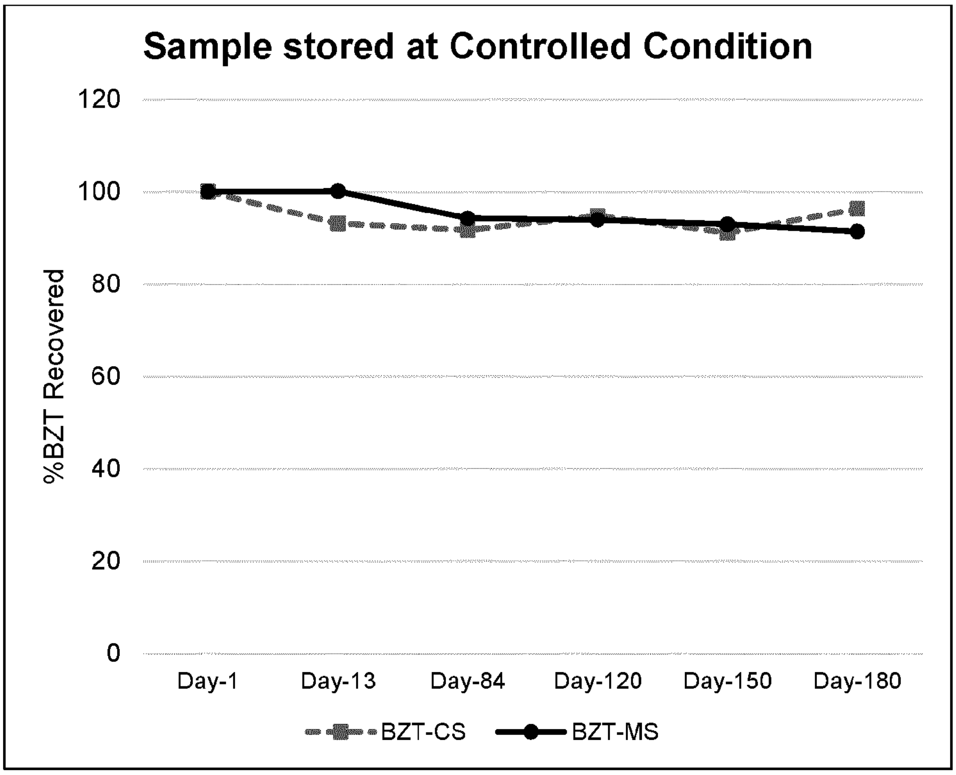
Sample stored at Controlled Condition
120
100
80
60
40
20
0
%BZT Recovered
Day-1
Day-13
Day-84
Day-120
Day-150
Day-180
BZT-CS
BZT-MS

FIG. 1B

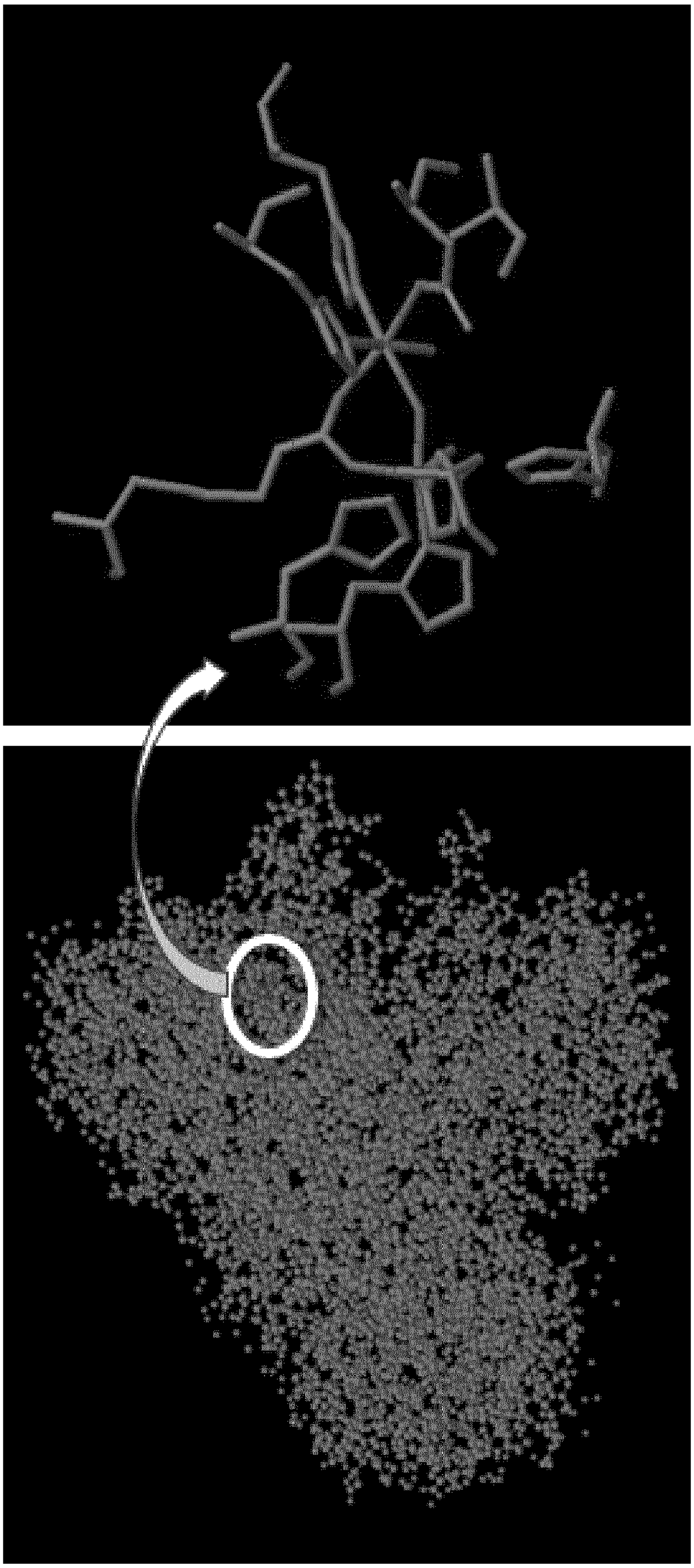

Sporosarcina Pasteurii Urease

FIG. 2A

Urease Active Site (optimized)

FIG. 2B

Europäisches Patentamt
European Patent Office
Office européen des brevets

# EUROPEAN SEARCH REPORT

Application Number
EP 21 21 2331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Pereira Araujo Débora ET AL: "Efficient sodium bisulfite-catalyzed synthesis of benzothiazoles and their potential as ureases inhibitors", RSC advances, 1 January 2015 (2015-01-01), pages 28814-28821, XP055925316, DOI: 10.1039/C5RA01081K Retrieved from the Internet: URL:https://pubs.rsc.org/en/content/articlepdf/2015/ra/c5ra01081k [retrieved on 2022-05-25] * abstract * ----- | 1-15 | INV. C05C9/00 C05C1/02 C05C3/00 C05C1/00 C05G3/90 C05G5/30 |
| A | TAHA MUHAMMAD ET AL: "Hybrid benzothiazole analogs as antiurease agent: Synthesis and molecular docking studies", BIOORGANIC CHEMISTRY, ACADEMIC PRESS INC., NEW YORK, NY, US, vol. 66, 24 March 2016 (2016-03-24), pages 80-87, XP029548417, ISSN: 0045-2068, DOI: 10.1016/J.BIOORG.2016.03.010 * abstract; table 1 * ----- | 1-15 | |
| | | | TECHNICAL FIELDS SEARCHED (IPC) |
| | | | C05C C05G |

The present search report has been drawn up for all claims

1

| Place of search | Date of completion of the search | Examiner |
|---|---|---|
| The Hague | 30 May 2022 | Rodriguez Fontao, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4530714 A, Kolc


### Non-patent literature cited in the description


- *Soil Use & Management,* 2008, vol. 24, 246
- *Soil Sci. Soc. Am. J.,* 2015
- *Soil Res.,* 2011, vol. 49, 315
- **CHRISTIANSON, C.B. et al.** *Soil Biology and Biochemistry,* 1993, 1107
- *RSC. Adv.,* 2015, vol. 5, 28814
- *J. Agric. Food Chem.,* 2008, vol. 56, 3721